# EUROPÄISCHE PATENTSCHRIFT

(11) **EP 0 221 493 B1**
(45) Veröffentlichungstag und Bekanntmachung des Hinweises auf die Patenterteilung: **25.05.1994**
(21) Anmeldenummer: 86114931.8
(22) Anmeldetag: 27.10.1986
(51) Int. Cl.: C12N 5/00

(54) **Verwendung von Gyrasehemmern zur Dekontamination von Mycoplasma-infizierten Zellkulturen**
Use of gyrase blocking agents in decontaminating cell cultures infested by mycoplasmas
Utilisation d'agents bloquant la gyrase pour la décontamination de cultures de cellules infestées par des mycoplasmes

(30) Priorität: 07.11.1985 DE 3539393; 27.05.1986 DE 3617803
(43) Veröffentlichungstag der Anmeldung: 13.05.1987
(73) Patentinhaber: BAYER AG, 51368 Leverkusen (DE)
(72) Erfinder: Hübner, Günter, Dr., D-5600 Wuppertal 1 (DE); Brunner, Helmut, Prof.Dr., D-4018 Langenfeld (DE); Zeiler, Hans-Joachim, Dr., 5600 Wuppertal 1 (DE)

(56) Entgegenhaltungen:
- FORTSCHRITTE DER ANTIMIKROBIELLEN UND ANTINEOPLASTISCHEN CHEMOTHERAPIE, Band 3, Nr. 5, 1984, Seiten 629-632; C. SIMON: "In-vitro-Aktivität von Gyrase-Hemmern gegen Mykoplasmen"
- JOURNAL ANTIMICROB. CHEMOTHER., Band 15, Nr. 6, Juni 1985, Seiten 787-789; R.J.FALLON et al.: "In-vitro sensitivity of legionellas, meningococci andmycoplasmas to ciprofloxacin and enoxacin"
- EUROPEAN JOURNAL OF CLINICAL MICROBIOLOGY, Band 3, Nr. 4, August 1984, Seiten 344-346; G.L. RIDGWAY et al.: "The activity of ciprofloxacin and other 4-quinolones against Chlamydia trachomatis and Mycoplasmas in vitro"
- ANTIMICROBIAL AGENTS AND CHEMOTHERAPY, Band 23, Nr. 5, Mai 1983, Seiten 641-648, American Society for Microbiology, US; S. NAKAMURA et al.: "In vitroantibacterial properties of AT-2266, a new pyridonecarboxylic acid"
- ANTIMCIROBIAL AGENTS AND CHEMOTHERAPY, Band 23, Nr. 3, März 1983, Seiten 509-511, American Society for Microbiology, US; Y. OSADA et al.: "Antimycoplasmal activity of ofloxacin (DL-8280)"
- JOURNAL OF CHROMATOGRAPHY, Band 339, Nr. 1, 1985, Seiten 214-218, Elsevier Science Publishers B.V., Amsterdam, NL; G. MONTAY et al.: "Improved high-performance liquid chromatographic determination of pefloxacin and its metabolite norfloxacin in human plasma and tissue"
- EUROPEAN JOURNAL OF CLINICAL MICROBIOLOGY, Band 2, Nr. 5, Oktober 1983, Seiten 479-480; C. SIMON et al.: "In vitro activity of norfloxacin against Mycoplasma hominis and Ureaplasma urealyticum"
- EXPERIMENTAL CELL RESEARCH, Band 152, 1984, Seiten 565-570, Academic Press,Inc.; J. SCHMIDT et al.: "Elimination of mycoplasmas from cell cultures and establishment of mycoplasma-free cell lines"
- JOURNAL OF IMMUNOLOGICAL METHODS, Band 109, Nr. 1, 1988, Seiten 17-25, Elsevier Science Publishers B.V. (Biomedical division); K. SCHMITT e tal.: "A safe and efficient method for elimination of cell culture mycoplasmas using ciprofloxacin"

## Beschreibung

Die Erfindung betrifft die Verwendung von Chinolon- und 1,8-Naphthyridon-3-carbonsäuren zur Dekontamination Mycoplasmen-infizierter Zellkulturen, Zubereitungen zur Dekontamination Mycoplasmen-infizierter Zellkulturen sowie Chinolon- und 1,8-Naphthyridon-3-carbonsäuren enthaltende Zellkulturmedien.

Zellkulturen, insbesondere permanente Zellinien, sind häufig mit Mycoplasmen kontaminiert, die viele unkontrollierbare Veränderungen in den Zellkulturen induzieren können. Im Gegensatz zu Infektionen mit Bakterien und Pilzen bleibt die Mycoplasmeninfektion oft unerkannt, weil selbst stark kontaminierte Zellkulturen normales Wachstum zeigen können und das Kulturmedium klar bleibt.

Für viele Zwecke der Grundlagenforschung (z.B. genetische und physiologische Gegebenheiten in den Zellen einer Kultur), der angewandten Forschung (z.B. lymphozytäre Zellfusion zur Gewinnung monoklonaler Antikörper) und erst recht der biotechnologischen Nutzung von Zellkulturen, z.B. Gewinnung eines Produktes für die Humanapplikation, ist es notwendig, mit Mycoplasma-freien Zellkulturen zu arbeiten. Ist eine Kultur erst einmal mit Mycoplasmen kontaminiert, so ist es äußerst schwer, wenn nicht gar oft unmöglich, diese Kultur wieder zu dekontaminieren, obwohl mit verschiedenen Techniken gelegentlich eine Dekontamination erreicht wurde: So wurden infizierte Zellkulturen bei 41°C für 18 h hyperthermisch behandelt (L. Hayflick, Nature 185, 783-784, 1960), oder die Zellen wurden mit Bromuracil bzw. Bromdesoxyuridin plus dem Fluorochrom Hoechst 33258 und UV-Licht behandelt (M. Marcus et al., Nature 285, 659-661, 1980; K. J. Fowler et al., Exp. Cell Res. 149, 303-306, 1983), oder sie wurden mit Makrophagen, ggf. plus Antibiotika inkubiert (L. Schimmelpfeng et al., Nature 285, 661-662, 1980), oder sie wurden durch die nackte Maus passagiert (D.N. Howell et al., Human Immunol. 5, 233-238, 1982). Für eine routinemäßige Dekontamination eignet sich jedoch keine dieser Methoden.

Es ist außerdem bekannt geworden, daß die Behandlung von Mykoplasmen-kontaminierten Zellkulturen und Zellinien mit den Antibiotika Tiamulin und Minocyclin zu Mykoplasmenfreien Kulturen führen soll. Um jedoch insbesondere bei wertvollen Zellinien endgültig Mykoplasmen-freie Kulturen zu etablieren, müssen verschiedene Schritte von Behandlung, Klonierung der Zellen und Kontrollen durchgeführt werden (J.Schmidt & V. Erfle, Exp. Cell Res. 152, 565 - 570, 1984).

Es wurde nun gefunden, daß Chinolon- und 1,8-Naphthyridon-3-carbonsäuren der Formel (I) 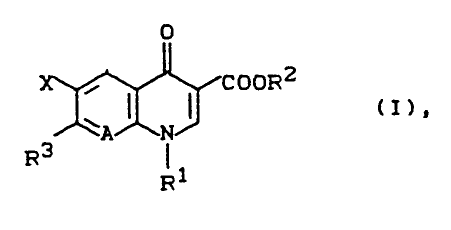
in welcher
- R¹: für Methyl, Ethyl, Propyl, Isopropyl, Cyclopropyl, Vinyl, 2-Hydroxyethyl, 2-Fluorethyl, Methoxy, Amino, Methylamino, Dimethylamino, Ethylamino, Phenyl, 4-Fluorphenyl, 2,4-Difluorphenyl,
- R²: für Wasserstoff, Alkyl mit 1 bis 4 Kohlenstoffatomen, (5-Methyl-2-oxo-1,3-dioxol-4-yl)-methyl,
- R³: für Methyl oder eine cyclische Aminogruppe wie

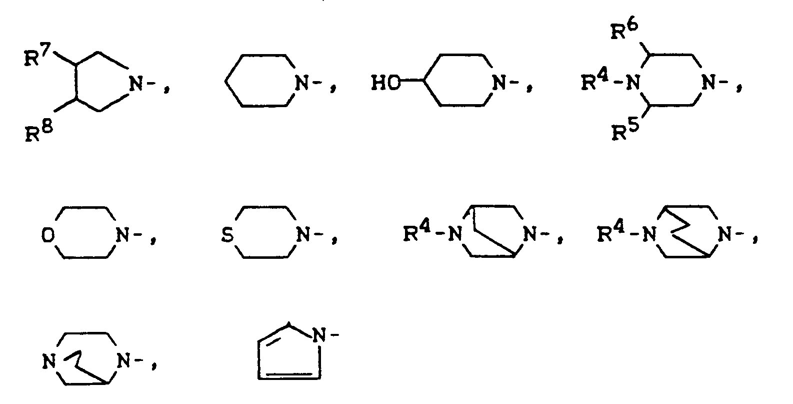

steht, worin.
- R⁴: für Wasserstoff, Alkyl mit 1 bis 4 Kohlenstoffatomen, 2-Hydroxethyl, Allyl, Propargyl, 2-Oxopropyl, 3-Oxobutyl, Phenacyl, Formyl, CFCl₂-S-, CFCl₂-SO₂-, CH₃O-CO-S-, Benzyl, 4-Aminobenzyl, 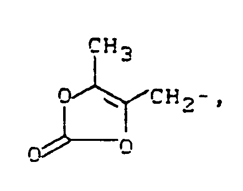
- R⁵: für Wasserstoff, Methyl,
- R⁶: für Wasserstoff, Alkyl mit 1 bis 4 Kohlenstoffatomen, Phenyl, Benzyloxmethyl,
- R⁷: für Wasserstoff, Amino, Methylamino, Ethylamino, Aminomethyl, Methylaminomethyl, Ethylaminomethyl, Dimethylaminomethyl, Hydroxy, Hydroxymethyl,
- R⁸: für Wasserstoff, Methyl, Ethyl oder Chlor steht,
- X: für Fluor, Chlor oder Nitro und
- A: für N oder C-R⁹ steht, worin
- R⁹: für Wasserstoff, Halogen wie Fluor oder Chlor, Methyl oder Nitro steht oder auch gemeinsam mit R¹ eine Brücke der Struktur 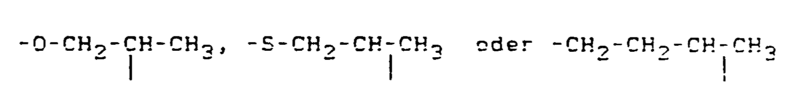 bilden kann,
bevorzugt in Konzentrationen zwischen 1 und 1000 µg/ml Zellkultur, besonders bevorzugt 20 bis 100 µg/ml Zellkultur, ganz besonders bevorzugt 50 µg/ml Zellkultur, bei der Behandlung von Zellkulturen, die gegebenenfalls gleichzeitig auch mit Tiamulin und Minocyclin inkubiert werden, eine Dekontamination besonders hartnäckiger Mycoplasmen-Infektionen erbringen, wie sie häufig in Kulturen adhärenter Zellen vorliegen, bei denen die Tiamulin- und Minocyclin-Behandlung nicht allein zum Erfolg führt. Das Verfahren benötigt nur eine siebentägige Behandlungszeit zur Erreichung einer dauerhaften Dekontamination der Kultur. Weitere verschiedene Schritte, wie z.B. Klonierung der Zellen, können völlig entfallen.

Zweckmäßigerweise werden zur Dekontamination 1 bis 1000 µg des Wirkstoffes pro ml Zellkultur eingesetzt.

Besonders bevorzugt werden für den erfindungsgemäßen Zweck Ciprofloxacin, Norfloxacin, Pefloxacin, Amifloxacin, Pirfloxacin, Piroxacin, Ofloxacin und Enoxacin eingesetzt.

Bevorzugt werden 1-Cyclopropyl-6-fluor-1,4-dihydro-4-oxo-7-piperazino-chinolin-3-carbonsäuren der Formel II 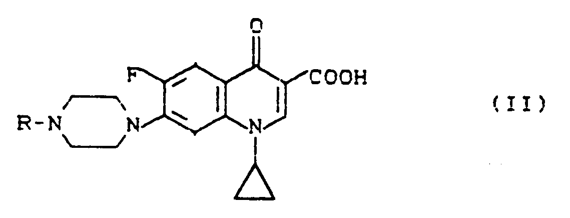
in der
- R: Wasserstoff, Methyl, Ethyl oder β-Hydroxyethyl bedeutet, ihre pharmazeutische verwendbaren Säureadditionssalze und Hydrate,
erfindungsgemäß verwendet.

Ganz besonders bevorzugt betrifft die Erfindung auch die Verwendung von 1-Cyclopropyl-6-fluor-1,4-dihydro-4-oxo-7-piperazinochinolin-3-carbonsäure und 1-Cyclopropyl-6-fluor-1,4-dihydro-4-oxo-7-(4-ethylpiperazino)-chinolin-3-carbonsäure sowie der Verbindungen der Beispiele zur Dekontamination von Mycoplasmen aus Zellkulturen derart, daß die Dekontamination mit der 1-Cyclopropyl-6-fluor-1,4-dihydro-4-oxo-7-piperazinochinolin-3-carbonsäure parallel zu einer Behandlung der Zellkulturen mit [(2-(Diethyl-amino)ethyl)-thio]-essigsäure-6-ethenyldecahydro-5-hydroxy-4,6,9,10-tetramethyl-1-oxo-3a,9-propano-3aH-cyclopentacycloocten-8-yl-ester = Tiamulin und/oder 4,7-Bis(dimethylamino)-1,4,4a,5,5a,6,11, 12a-octahydro-3,10,12,12a-tetrahydroxy-1,11-dioxo-2-naphthacencarbonsäureamid = Minocyclin erfolgt. Dabei werden 1 bis 1000 µg Tiamulin und/oder Minocyclin pro ml Zellkultur eingesetzt. Die Behandlung erfolgt so, daß man den Wirkstoff 1-Cyclopropyl-6-fluor-1,4-dihydro-4-oxo-7-piperazinochinolin-3-carbonsäure oder 1-Cyclopropyl-6-fluor-1,4-dihydro-4-oxo-7-(4-ethylpiperazino)-chinolin-3-carbonsäure 7 bis 14 Tage, gegebenenfalls in Kombination mit den Wirkstoffen Tiamulin und/oder Minocyclin, auf die Zellkultur einwirken läßt.

Weiterer Gegenstand der Erfindung ist auch die Verwendung von Chinolon- und 1,8-Naphthyridon-3-carbonsäuren der Formeln (I) und (II) für die Herstellung von Zubereitungen zur Dekontamination von Mycoplasma-infizierten Zellkulturen sowie insbesondere die Verwendung von 1-Cyclopropyl-6-fluor-1,4-dihydro-4-oxo-7-piperazinochinolin-3-carbonsäure, gegebenenfalls in Verbindung mit Tiamulin und/oder Minocyclin für die Herstellung solcher Zubereitungen.

Schließlich sind auch Zellkulturmedien, enthaltend Chinolon- und 1,8-Naphthyridon-3-carbonsäuren der Formeln (I) und (II) bzw. Zellkulturen, enthaltend Chinolon- und Naphthyridon-3-carbonsäuren der Formeln (I) und (II) und gegebenenfalls Tiamulin und/oder Minocyclin Gegenstand der Erfindung.

Zweckmäßigerweise bietet es sich an, nach der Detektion einer Mycoplasmeninfektion in Zellkulturen, insbesondere in wertvollen Zellinien, diese Zellen in einem geeigneten Kulturmedium in einer Dichte von z.B. 10³ - 10⁶ Zellen/ml frisch einzusäen in ein geeignetes Kulturgefäß wie beispielsweise eine Zellkulturflasche aus Plastikmaterial. Diese frisch inokulierten Zellen können dann mit einer zuvor hergestellten Lösung von beispielsweise Ciprofloxacin, gegebenenfalls auch mit einer Lösung von Tiamulin und/oder Minocyclin versetzt werden. Das Antibiotikum kann beispielsweise im Zellkulturmedium gelöst und in einem nach Bedarf erforderlichen Volumen zur Zellkultur zugegeben werden; so ist es z.B. praktisch, etwa 100 -500 µl Antibiotika-Lösung zu etwa 5 - 20 ml Zellkultur zuzupipettieren. Nach Zell-und Antibiotika-Inokulation wachsen die Zellen in den nachfolgenden Tagen an; während dieser Zeit kann dann Ciprofloxacin, gegebenenfalls auch Tiamulin und/oder Minocyclin, seine antiinfektive Wirkung auf die Mycoplasmen ausüben. Ist die Kultur dicht angewachsen (stationäre Wachstumsphase), so empfiehlt es sich, die Zellen mit einer dem Fachmann bekannten Technik zu ernten und mit einer niedrigeren Zelldichte wieder frisch in ein neues Kulturgefäß zu inokulieren; dazu sollte frisches Kulturmedium verwendet werden und bei Bedarf wieder Ciprofloxacin-Lösung, gegebenenfalls auch Tiamulin-und/oder Minocyclin-Lösung zugesetzt werden.

### Beispiel 1

Suspensions- oder adhärente Zellen verschiedener Zellinien (z.B. U 266 humane Myelomzellen, L 929 Maus-Fibroblasten), die mit Mycoplasmen nicht identifizierter Spezifität kontaminiert waren, wurden mit einer Konzentration von 10^5/ml und einem Volumen von 10-15 ml in 75 cm³-Zellkulturflaschen eingesät. Als Kulturmedium wurde RPMI 1640 mit 10% fötalem Kälberserum (FCS), L-Glutamin (2 mM), Hepes-Puffer (4 mM) und 2-Mercaptoethanol (50 µM) verwandt. Nach jeweils 3-4 Tagen befanden sich die Kulturen in stationärer Phase, in der sie dann geerntet wurden (bei adhärenten Zellen unter Verwendung von Trypsin/EDTA [0,25%/0,02%]). Anschließend wurden die Zellen wieder in frischem Medium bei 10⁵/ml ausgesät.

Zur Behandlung der Mycoplasmen-Kontamination wurde den Kulturen zugesetzt: a) Tiamulin (10 µg/ml, 4 Tage lang) und anschließend Minocyclin (5 ug/ml, 3 Tage lang); b) Ciprofloxacin (50 µg/ml, 7 Tage lang); c) Tiamulin und Minocyclin wie unter a) plus Ciprofloxacin wie unter b). Am Ende jeder Einwirkzeit wurden die Antibiotika mit Medium ausgewaschen und die Zellen weiterpassagiert. Vor der Behandlung und zu verschiedenen Zeiten nach der Behandlung wurde 0,1 ml Zellkultur auf Hayflick's Agar zum Mycoplasmen-Nachweis ausgestrichen (L. Hayflick, Texas Reports Biol. Med. 23, Suppl. 1, 285-300, 1965) und für mindestens eine Woche bei 37°C anaerob bebrütet.

Das verwendete Agarmedium (5 ml pro Petrischale) setzte sich zusammen aus: PPLO-Agar (Difco; 70 Teile), Pferdeserum (20 Teile), Hefeextrakt (Flow; 25%-ig; 10 Teile), Glucose (50%-ig, 2 Teile), Benzylpenicillin (100000 U/ml; 1 Teil) und Thallium-I-acetat (2%-ig; 2,5 Teile). Die Entstehung typischer "spiegeleiförmiger" Kolonien auf dem Agar bedeutet Kontamination der Zellkultur mit Mycoplasmen. Tabelle 1 zeigt Ergebnisse von behandelten L 929-Zellkulturen.

**Tabelle 1**

| Mycoplasmen-Kontamination in L 929-Zellkulturen zu verschiedenen Zeiten nach einer 7-tägigen Behandlung mit Tiamulin/Minocyclin(T/M), Ciprofloxacin (C), Tiamulin-Ciprofloxacin/Minocyclin-Ciprofloxacin (T-C/M-C) oder ohne Behandlung (ø); + = Mycoplasmen-Kontamination, - = keine Mycoplasmen-Kontamination | | | | | | | |
|---|---|---|---|---|---|---|---|
| Tage | 0 | 4 | 7 | 14 | 21 | 31 | 36 |
| T/M | - | + | + | + | + | + | + |
| C | - | - | - | + | + | + | + |
| T-C/M-C | - | - | - | - | - | - | - |
| ø | + | + | + | + | + | + | + |

Während sofort nach der Behandlung in keiner der drei Behandlungsgruppen Mycoplasmen nachweisbar waren, konnten 4 Tage nach Absetzen der Tiamulin/Minocyclin-Behandlung wieder Mycoplasmen aufgefunden werden, nach Ciprofloxacin-Behandlung konnten 7 Tage lang keine Mycoplasmen entdeckt werden. Im Gegensatz dazu konnten über einen Monat nach der Kombinationsbehandlung mit Tiamulin-Ciprofloxacin/Minocyclin-Ciprofloxacin keine Mycoplasmen mehr nachgewiesen werden, so daß diese Zellen als dekontaminiert angesehen werden müssen.

### Beispiel 2

Als Wirkstoff wurde 1-Cyclopropyl-6-fluor-1,4-dihydro-4-oxo-7-(4-ethylpiperazino)-chinolin-3-carbonsäure verwendet, die im folgenden mit dem Großbuchstaben A bezeichnet wird.

Suspensions- oder adhärente Zellen verschiedener Zelllinien (z.B. U 266 humane Myelomzellen, L 929-Fibroblasten), die mit Mycoplasmen nicht identifizierter Spezifität kontaminiert waren, wurden mit einer Konzentration von 10⁵ Zellen/ml und einem Volumen von 10-15 ml in 75 cm³ -Zellkulturflaschen eingesät. Als Kulturmedium wurde RPMI 1640 mit 10 % fötalem Kälberserum (FCS), L-Glutamin (2mM) und Hepes-Puffer (4 mM) verwandt. Nach jeweils 3-4 Tagen befanden sich die Kulturen in stationärer Phase, in der sie dann geerntet wurden (bei, adhärenten Zellen unter Verwendung von Trypsin/EDTA 0,25 %/0,02 %). Anschließend wurden die Zellen wieder in frischem Medium bei 10⁵ Zellen/ml ausgesät.

Zur Behandlung der Mycoplasmen-Kontamination wurde den Kulturen zugesetzt: a) A (50 µg/ml, 7 Tage lang); b) A (10 µg/ml, 4 Tage lang) plus Tiamulin (10 µg/ml, 4 Tage lang)und anschließend A (10 µg/ml), 3 Tage lang) plus Minocyclin (5 µg/ml, 3 Tage lang). Am Ende jeder Einwirkzeit wurden die Antibiotika mit Medium ausgewaschen und die Zellen weiterpassagiert. Vor der Behandlung und zu verschiedenen Zeiten nach der Behandlung wurde 0,1 ml Zellkultur auf Hayflick's Agar zum Mycoplasmen-Nachweis ausgestrichen (L. Hayflick, Texas Reports Biol. Med. 23, Suppl. 1, 285-300, 1965] und für mindestens eine Woche bei 37°C anaerob bebrütet.

Das verwendete Agarmedium (5 ml pro Petrischale) setzte sich zusammen aus: PPLO-Agar (Difco; 70 Teile), Pferdeserum (20 Teile), Hefeextrakt (Flow; 25 %ig; 10 Teile), Glucose (50 %ig, 2 Teile), Benzylpenicillin (100 000 U/ml; 1 Teil) und Thallium-I-acetat (2 %ig, 2,5 Teile). Die Entstehung typischer "spiegeleiförmiger" Kolonien auf dem Agar bedeutet Kontamination der Zellkultur mit Mycoplasmen. Tabelle 2 zeigt Ergebnisse von behandelten U 266-Zellkulturen.

**Tabelle 2**

| Mycoplasma-Kontamination in U 266-Zellkulturen zu verschiedenen Zeiten nach einer 7 tägigen Behandlung mit 50 µg/ml A, oder 10 µg/ml A zusammen mit 10 µg/ml Tiamulin für 4 Tage und anschließend zusammen mit 5 µg/ml Minocyclin für 3 Tage (T-A/M-A) oder ohne Behandlung (ø); + = Mycoplasma-Kontamination, - = keine Mycoplasma-Kontamination | | | | |
|---|---|---|---|---|
| Tage | 0 | 21 | 28 | 35 |
| A | - | - | - | - |
| T-A/M-A | - | - | - | - |
| ø | + | + | + | + |

Im Gegensatz zur unbehandelten Kontrolle konnten in beiden Behandlungsgruppen über einen Monat nach Behandlungsende keine Mycoplasmen nachgewiesen werden, Diese Zellen müssen somit als dekontaminiert angesehen werden,

### Beispiel 3

Die Kultur- und Testbedingungen in diesem Beispiel sind die gleichen wie im 2. Beispiel. Hier wurden lediglich Chinolone in U 266-Zellkulturen eingesetzt, ohne daß Tiamulin und Minocyclin verwendet wurden.

Es wurden 8-Chlor-1-cyclopropyl-6-fluor-1,4-dihdyro-4-oxo-7-(3-methyl-1-piperazinyl)-3-chinolincarbonsäure = B, 8-Chlor-1-cyclopropyl-6-fluor-1,4-dihydro-4-oxo-7-(1-piperazinyl)-3-chinolincarbonsäure = D und 8-Chlor-1-cyclopropyl-6-fluor-7-methyl-1,4-dihydro-5-oxo-3-chinolin-carbonsäure = E eingesetzt.

**Tabelle 3**

| Mycoplasma-Kontamination in U 266-Zellkulturen zu verschiedenen Zeiten nach einer 7-tägigen Behandlung mit jeweils 10 µg/ml der Substanzen B (B), oder D (D), oder E (E) gelöst in Ethanol plus Beschallung, Ethanol-Konzentration in Kultur 0,25 %), oder ohne Behandlung ø); + = Mycoplasma-Kontamination, - = keine Mycoplasma-Kontamination | | | | |
|---|---|---|---|---|
| Tage | 0 | 21 | 28 | 35 |
| B | - | - | - | - |
| D | - | - | - | - |
| E | - | - | - | - |
| ø | + | + | + | + |

Im Gegensatz zur unbehandelten Kontrolle waren alle Behandlungen erfolgreich, so daß über einen Monat nach Behandlungsende keine Mycoplasma-Kontamination nachweibar war. Diese Kulturen müssen deshalb als dekontaminiert angesehen werden. Zuletzt soll noch darauf hingewiesen werden, daß die Substanzen D und E leichte toxische Effekte auf die Kulturen ausübten, d.h. die Zellen waren während der Behandlung in ihrer Proliferation gehemmt, ohne daß sich nach Behandlungsende eine Verminderung der Proliferationsfähigkeit der Zellen bemerkbar machte.

## Patentansprüche

1. Verwendung von Chinolon- und 1,8-Naphthyridoncarbonsäuren der Formel (I) 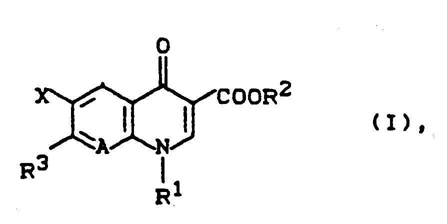 in welcher
R¹ für Methyl, Ethyl, Propyl, Isopropyl, Cyclopropyl, Vinyl, 2-Hydroxyethyl, 2-Fluorethyl, Methoxy, Amino, Methylamino, Dimethylamino, Ethylamino, Phenyl, 4-Fluorphenyl, 2,4-Difluorphenyl,
R² für Wasserstoff, Alkyl mit 1 bis 4 Kohlenstoffatomen, (5-Methyl-2-oxo-1,3-dioxol-4-yl)-methyl,
R³ für Methyl oder eine cyclische Aminogruppe wie 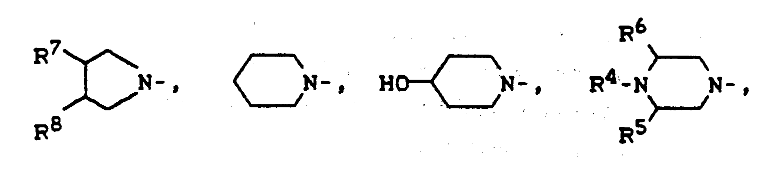 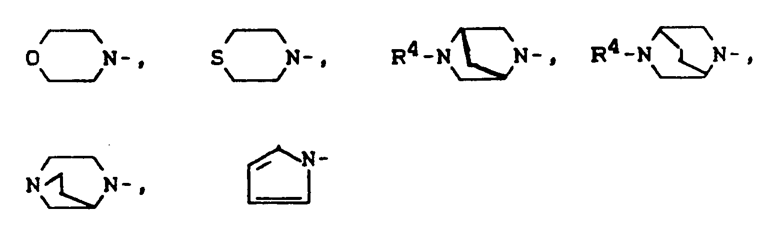
steht, worin
R⁴ für Wasserstoff, Alkyl mit 1 bis 4 Kohlenstoffatomen, 2-Hydroxethyl, Allyl, Propargyl, 2-Oxopropyl,3-Oxobutyl, Phenacyl, Formyl, CFCl₂-S-, CFCl₂-SO₂-, CH₃O-CO-S-, Benzyl, 4-Aminobenzyl, 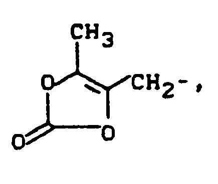
R5 für Wasserstoff, Methyl,
R6 für Wasserstoff, Alkyl mit 1 bis 4 Kohlenstoffatomen, Phenyl, Benzyloxmethyl,
R⁷ für Wasserstoff, Amino, Methylamino, Ethylamino, Aminomethyl, Methylaminomethyl, Ethylaminomethyl, Dimethylaminomethyl, Hydroxy, Hydroxymethyl,
R⁸ für Wasserstoff, Methyl, Ethyl oder Chlor steht,
X für Fluor, Chlor oder Nitro und
A für N oder C-R⁹ steht, worin
R⁹ für Wasserstoff, Halogen wie Fluor oder Chlor, Methyl oder Nitro steht oder auch gemeinsam mit R¹ eine Brücke der Struktur 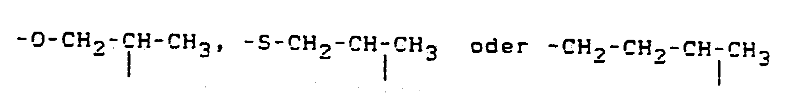 bilden kann,
zur Dekontamination von Mycoplasma-infizierten Zellkulturen.

2. Verwendung von Verbindungen der Formel I gemäß Anspruch 1 zur Dekontamination von Mycoplasma-infizierten Zellkulturen, dadurch gekennzeichnet, daß zur Dekontamination 1 bis 1000 µg/ml Zellkultur eingesetzt werden.

3. Verwendung von 1-Cyclopropyl-6-fluor-1,4-dihydro-4-oxo-7-piperazino-chinolin-3-carbonsäuren der Formel II 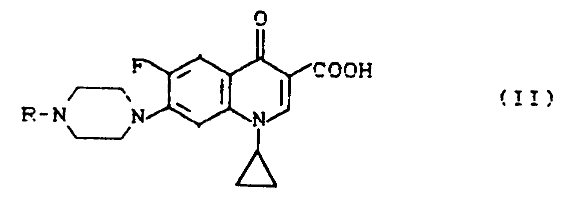 in der
R Wasserstoff, Methyl, Ethyl oder Hydroxyethyl bedeutet,
und von ihren pharmazeutisch verwendbaren Säureadditionssalzen und Hydraten zur Dekontamination von Mykoplasmen-infizierten Zellkulturen.

4. Verwendung von 1-Cyclopropyl-6-fluor-1,4-dihydro-4-oxo-7-piperazino-chinolin-3-carbonsäure zur Dekontamination von Mycoplasma-infizierten Zellkulturen, dadurch gekennzeichnet, daß zur Dekontamination 1 bis 1000 µg/ml Zellkultur eingesetzt werden.

5. Verwendung von Verbindungen der Formel I bzw. II gemäß Anspruch 1 bzw. 3 zur Dekontamination von Mycoplasma-infizierten Zellkulturen, dadurch gekennzeichnet, daß die Dekontamination mit den Verbindungen der Formel I parallel zu einer Behandlung der Zellkulturen mit [(2-(Diethylamino)ethyl)-thio]-essigsäure-6-ethenyldecahydro-5-hydroxy-4,6,9,10-tetramethyl-1-oxo-3a,9-propano-3aH-cyclopentacycloocten-8-yl-ester = Tiamulin und/oder 4,7-Bis(dimethylamino)-1,4,4a,5,5a,6,11,12a-octahydro-3,10,12, 12a-tetrahydroxy-1,11-dioxo-2-naphthacencarbonsäureamid = Minocyclin erfolgt.

6. Verwendung von Verbindungen der Formel I bzw. II gemäß Anspruch 1 bzw. 3, dadurch gekennzeichnet, daß zur Dekontamination 1 bis 1000 µg der Verbindungen der Formel I und 1 bis 1000 µg Tiamulin und/oder Minocyclin pro ml Zellkultur eingesetzt werden.

7. Verwendung von Verbindungen der Formel I bzw. II gemäß den Ansprüchen 1 bis 6, dadurch gekennzeichnet, daß man die Verbindungen der Formel I 7 bis 14 Tage auf die Zellkultur einwirken läßt.

8. Verwendung von Verbindungen der Formel I bzw. II gemäß den Ansprüchen 1 bis 7, dadurch gekennzeichnet, daß man die Verbindungen der Formel I 7 bis 14 Tage, in Kombination mit den Wirkstoffen Tiamulin und/oder Minocyclin auf die Zellkultur einwirken läßt.

9. Verwendung von Verbindungen der Formel I bzw. II gemäß Anspruch 1 bzw. 3 für die Herstellung von Zubereitungen zur Dekontamination von Mycoplasmainfizierten Zellkulturen.

10. Verwendung gemäß Anspruch 8 in Verbindung mit Tiamulin und/oder Minocyclin für die Herstellung von Zubereitungen zur Dekontamination von Mycoplasmainfizierten Zellkulturen.

11. Verwendung gemäß den Ansprüchen 1 bis 10, dadurch gekennzeichnet, daß man 1-Cyclopropyl-6-fluor-1,4-dihydro-4-oxo-7-piperazinochinolin-3-carbonsäure und/oder 1-Cyclopropyl-6-fluor-1,4-dihydro-4-oxo-7-(4-ethyl-piperazino)-chinolin-3-carbonsäuren verwendet.

12. Zellkulturmedien, enthaltend Verbindungen der Formel I bzw. II nach Anspruch 1 bzw. 3.

13. Zellkulturmedien, enthaltend Verbindungen der Formel I bzw. II nach Anspruch 1 bzw. 3 und Tiamulin und/oder Minocyclin.

## Claims

1. Use of quinolonecarboxylic acids and 1,8-naphthyridonecarboxylic acids of the formula (I) 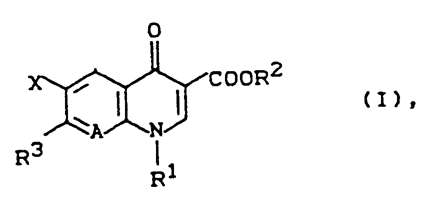 in which
R¹ represents methyl, ethyl, propyl, isopropyl, cyclopropyl, vinyl, 2-hydroxyethyl, 2-fluoroethyl, methoxy, amino, methylamino, dimethylamino, ethylamino, phenyl, 4-fluorophenyl or 2,4-difluorophenyl,
R² represents hydrogen, alkyl having 1 to 4 carbon atoms, or (5-methyl-2-oxo-1,3-dioxol-4-yl)-methyl,
R³ represents methyl, or a cyclic amino group such as 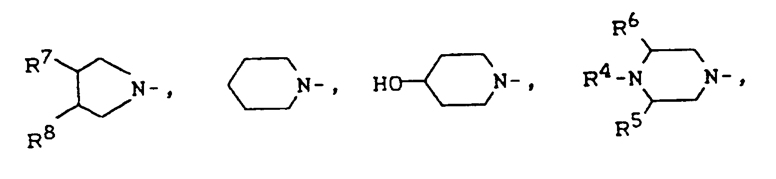 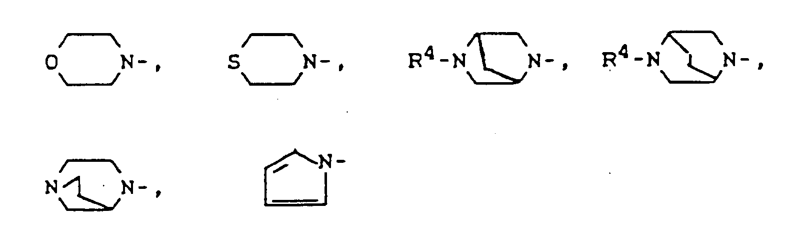
in which
R⁴ represents hydrogen, alkyl having 1 to 4 carbon atoms, 2-hydroxyethyl, allyl, propargyl, 2-oxopropyl, 3-oxobutyl, phenacyl, formyl, CFCl₂-S-, CFCl₂-SO₂-, CH₃O-CO-S-, benzyl, 4-aminobenzyl or 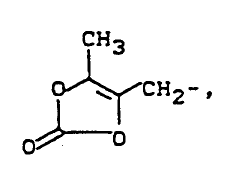
R⁵ represents hydrogen or methyl,
R⁶ represents hydrogen, alkyl having 1 to 4 carbon atoms, phenyl or benzyloxymethyl,
R⁷ represents hydrogen, amino, methylamino, ethylamino, aminomethyl, methylaminomethyl, ethylaminomethyl, dimethylaminomethyl, hydroxyl or hydroxymethyl,
R⁸ represents hydrogen, methyl, ethyl or chlorine,
X represents fluorine, chlorine or nitro, and
A represents N or C-R⁹, where
R⁹ represents hydrogen, halogen such as fluorine or chlorine, methyl or nitro, or, together with R¹, can form a bridge of the structure 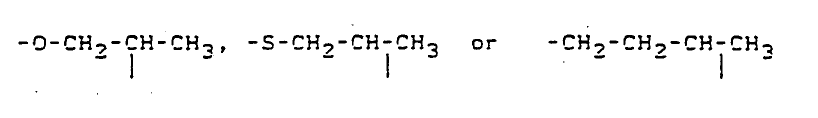
for decontaminating mycoplasma-infected cell cultures.

2. Use of compounds of the formula I according to Claim 1 for decontaminating mycoplasma-infected cell cultures, characterized in that, for the decontamination, 1 to 1,000 µg are employed per ml of cell culture.

3. Use of 1-cyclopropyl-6-fluoro-1,4-dihydro-4-oxo-7-piperazino-quinoline-3-carboxylic acids of the formula II 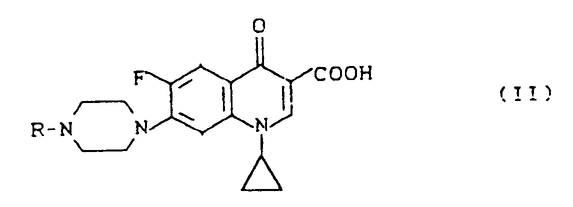 in which
R denotes hydrogen, methyl, ethyl or hydroxyethyl,
and of their pharmaceutically utilizable acid addition salts and hydrates, for decontaminating mycoplasma-infected cell cultures.

4. Use of 1-cyclopropyl-6-fluoro-1,4-dihydro-4-oxo-7-piperazino-quinoline-3-carboxylic acid for decontaminating mycoplasma-infected cell cultures, characterized in that, for the decontamination, 1 to 1,000 µg are employed per ml of cell culture.

5. Use of compounds of the formula I or II according to Claim 1 or 3, respectively, for decontaminating mycoplasma-infected cell cultures, characterized in that the decontamination with the compounds of the formula I is effected in parallel with a treatment of the cell cultures with [(2-(diethylamino)ethyl)thio]-acetic acid 6-ethenyldecahydro-5-hydroxy-4,6,9,10-tetramethyl-1-oxo-3a,9-propano-3aH-cyclopentacycloocten-8-yl ester = tiamulin and/or 4,7-bis(dimethylamino)-1,4,4a,5,5a,6,11,12a-octahydro-3,10,12,12a-tetrahydroxy-1,11-dioxo-2-naphthacene-carboxamide = minocycline.

6. Use of compounds of the formula I or II according to Claim 1 or 3, respectively, characterized in that, for the decontamination, 1 to 1,000 µg of the compounds of the formula I and 1 to 1,000 µg of tiamuli and/or minocycline are employed per ml of cell culture.

7. Use of compounds of the formula I or II according to Claims 1 to 6, characterized in that the compounds of the formula I are allowed to act on the cell culture for 7 to 14 days.

8. Use of compounds of the formula I or II according to Claims 1 to 7, characterized in that the compounds of the formula I are allowed to act on the cell culture for 7 to 14 days in combination with the active compounds tiamulin and/or minocycline.

9. Use of compounds of the formula I or II according to Claim 1 or 3, respectively, for producing preparations for decontaminating mycoplasma-infected cell cultures.

10. Use according to Claim 8 in combination with tiamulin and/or minocycline for producing preparations for decontaminating mycoplasma-infected cell cultures.

11. Use according to Claims 1 to 10, characterized in that 1-cyclopropyl-6-fluoro-1,4-dihydro-4-oxo-7-piperazinoquinoline-3-carboxylic acid and/or 1-cyclopropyl-6-fluoro-1,4-dihydro-4-oxo-7-(4-ethylpiperazino)-quinoline-3-carboxylic acids are used.

12. Cell culture media, containing compounds of the formula I or II according to Claim 1 or 3, respectively.

13. Cell culture media, containing compounds of the formula I or II according to Claim 1 or 3, respectively, and tiamulin and/or minocycline.

## Revendications

1. Utilisation d'acides quinolone- et 1,8-naphtyridone-carboxyliques de formule (I) 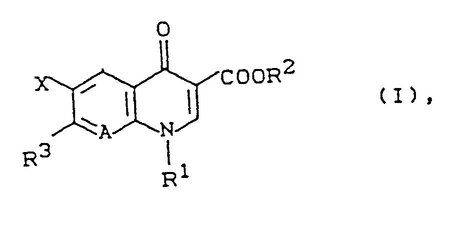 dans laquelle
R¹ représente un groupe méthyle, un groupe éthyle, un groupe propyle, un groupe isopropyle, un groupe cyclopropyle, un groupe vinyle, un groupe 2-hydroxyéthyle, un groupe 2-fluoréthyle, un groupe méthoxy, un groupe amino, un groupe méthylamino, un groupe diméthylamino, un groupe éthylamino, un groupe phényle, un groupe 4-fluorophényle, un groupe 2,4-difluorophényle,
R² représente un atome d'hydrogène, un groupe alkyle contenant de 1 à 4 atomes de carbone, un groupe (5-méthyl-2-oxo-1,3-dioxol-4-yl)-méthyle,
R³ représente un groupe méthyle ou un groupe amino cyclique tel que 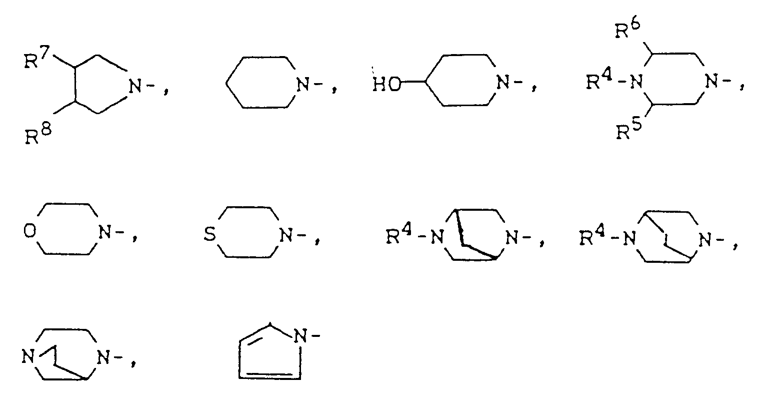
où
R⁴ représente un atome d'hydrogène, un groupe alkyle contenant de 1 à 4 atomes de carbone, un groupe 2-hydroxyéthyle, un groupe allyle, un groupe propargyle, un groupe 2-oxopropyle, un groupe 3-oxobutyle, un groupe phénacyle, un groupe formyle, CFCl₂-S-, CFCl₂-SO₂-, CH₃O-CO-S-, un groupe benzyle, un groupe 4-aminobenzyle, 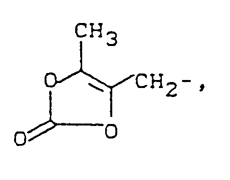
R⁵ représente un atome d'hydrogène, un groupe méthyle,
R⁶ représente un atome d'hydrogène, un groupe alkyle contenant de 1 à 4 atomes de carbone, un groupe phényle, un groupe benzyloxyméthyle,
R⁷ représente un atome d'hydrogène, un groupe amino, un groupe méthylamino, un groupe éthylamino, un groupe aminométhyle, un groupe méthylaminométhyle, un groupe éthylamino-méthyle, un groupe diméthylaminométhyle, un groupe hydroxyle, un groupe hydroxyméthyle,
R⁸ représente un atome d'hydrogène, un groupe méthyle, un groupe éthyle ou un atome de chlore,
X représente un atome de fluor, un atome de chlore ou un groupe nitro, et
A représente N ou C-R⁹ où
R⁹ représente un atome d'hydrogène, un atome d'halogène tel qu'un atome de fluor ou un atome de chlore, un groupe méthyle ou un groupe nitro, ou ensemble avec R¹, peut former un pont de structure 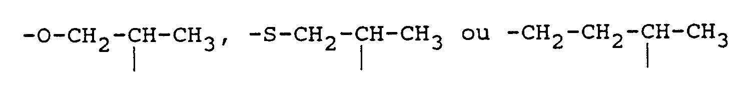
pour la décontamination de cultures cellulaires infectées par des mycoplasmes.

2. Utilisation de composés de formule I selon la revendication 1 pour la décontamination de cultures cellulaires infectées par des mycoplasmes, qui se caractérise par la mise en oeuvre de 1 à 1000 µg/ml de culture cellulaire à des fins de décontamination.

3. Utilisation d'acides 1-cyclopropyl-6-fluoro-1,4-dihydro-4-oxo-7-pipérazinoquinoléine-3-carboxyliques de formule II 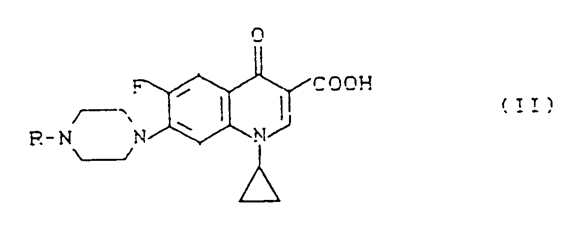 dans laquelle
R représente un atome d'hydrogène, un groupe méthyle, un groupe éthyle ou un groupe hydroxyéthyle,
et de leurs sels d'addition d'acides et hydrates pharmaceutiquement utilisables pour la décontamination de cultures cellulaires infectées par des mycoplasmes.

4. Utilisation d'acide 1-cyclopropyl-6-fluoro-1,4-dihydro-4-oxo-7-pipérazinoquinoléine-3-carboxylique pour la décontamination de cultures cellulaires infectées par des mycoplasmes, qui se caractérise par la mise en oeuvre de 1 à 1000 µg/ml de culture cellulaire à des fins de décontamination.

5. Utilisation de composés des formules I ou II selon les revendications 1 ou 3 pour la décontamination de cultures cellulaires infectées par des mycoplasmes, qui se caractérise par le fait que la décontamination a lieu avec les composés de formule I parallèlement à un traitement des cultures cellulaires avec l'ester 6-éthényldécahydro-5-hydroxy-4,6,9,10-tétraméthyl-1-oxo-3a,9-propano-3aH-cyclopentacyclo-octén-8-ylique de l'acide [(2-(diéthylamino)éthyl)-thio]acétique = tiamuline et/ou de l'amide de l'acide 4,7-bis(diméthylamino)-1,4,4a,5,5a,6,11,12a-octahydro-3,10,12,12a-tétrahydroxy-1,11-dioxo-2-naphtacènecarboxylique = minocycline.

6. Utilisation de composés des formules I ou II selon les revendications 1 ou 3, caractérisée en ce que, à des fins de décontamination, on met en oeuvre de 1 à 1000 µg des composés de formules I et II et de 1 à 1000 µg de tiamuline et/ou de minocycline par ml de culture cellulaire.

7. Utilisation des composés de formule I ou II selon les revendications 1 à 6, caractérisé en ce qu'on laisse agir les composés de formule I de 7 à 14 jours sur la culture cellulaire.

8. Utilisation des composés de formule I ou II selon les revendications 1 à 7, caractérisée en ce qu'on laisse agir les composés de formule I de 7 à 14 jours en combinaison avec les substances actives tiamuline et/ou minocycline sur la culture cellulaire.

9. Utilisation de composés de formule I ou II selon les revendications 1 ou 3 pour la mise au point de préparations à des fins de décontamination de cultures cellulaires infectées par des mycoplasmes.

10. Utilisation selon la revendication 8, en liaison avec la tiamuline et/ou la minocycline pour la mise au point de préparation à des fins de décontamination de cultures cellulaires infectées par des mycoplasmes.

11. Utilisation selon les revendications 1 à 10, caractérisée en ce qu'on utilise l'acide 1-cyclopropyl-6-fluoro-1,4-dihydro-4-oxo-7-pipérazinoquinoléine-3-carboxylique et/ou l'acide 1-cyclopropyl-6-fluoro-1,4-dihydro-4-oxo-7-(4-éthylpipérazino)-quinoléine-3-carboxylique.

12. Milieux de cultures cellulaires contenant des composés de formule I ou II selon les revendications 1 ou 3.

13. Milieux de cultures cellulaires contenant des composés des formules I ou II selon les revendications 1 ou 3, ainsi que de la tiamuline et/ou de la minocycline.
